# EUROPEAN PATENT SPECIFICATION

(11) **EP 2 145 566 B1**
(45) Date of publication and mention of the grant of the patent: **27.04.2011**
(21) Application number: 08252448.9
(22) Date of filing: 17.07.2008
(51) Int. Cl.: A47C 7/00, A61M 5/14, F16M 11/28

(54) **Intraveneous support apparatus**
Intravenöse Stützvorrichtung
Appareil de support intraveineux

(43) Date of publication of application: 20.01.2010
(73) Proprietor: AMG Medical Inc., Montreal QC H4T 1V5 (CA)
(72) Inventor: Meyers, Daniel Seth, Roxboro, Quebec H8Y 3A6 (CA); Boar, Cristian, Montreal, Quebec H9B 2G7 (CA); Xu, Ming, Saint-Laurent, Quebec H4R 3N4 (CA)
(74) Representative: Leckey, David Herbert

(56) References cited:
- EP-A- 0 051 732
- EP-A- 1 212 963
- US-A- 1 261 755
- US-A- 3 863 876
- US-A- 4 332 378
- US-A- 4 821 986

## Description

### FIELD OF THE INVENTION

The present invention relates generally to the field of medical equipment and, more particularly, to an intravenous support apparatus to hang an intravenous liquid supply in order to enable gravity flowing of the intravenous liquids to a patient.

### BACKGROUND OF THE INVENTION

Intravenous support apparatuses which are usually referred to as I.V. stands or I.V. poles, are conventionally constructed having a tall slender upright post or pole mounted onto a relatively small-sized base, sometimes with small wheels. The post or pole is fitted with a hanger at the top from which the bottles or pouches of intravenous liquids are hung. Optionally, an I.V. pump may be supported on the post or pole and connected to the bottles or pouches to pump liquids. For convenience of use, I.V. stands are sometimes configured having the post or pole in a telescoping configuration in which the sections of the post or pole have a diameter smaller one than another from a bottom section to a top section, in order to allow adjustment of the height level of the bottles or pouches of the intravenous liquids for controlling the gravity flowing of the liquids and for convenience of access.

US-4332378 discloses an ambulatory patient support stand. US-4821986 discloses a muliple leg furniture base. EP-1212963 discloses a base for tables. US-1261755 discloses an embalming stand. US-3863876 discloses an upright support. EP-0051732 discloses a star-shaped pedestal.

Also for convenience of use, the base of stands are usually relatively small. However, even under the regular load of the weight of the bottles or plastic pouches containing liquids, which are hung on the top of the post or pole, the I.V. stands are relatively unstable and can be rather easily knocked over. Therefore it is not unusual for the conventional I.V. stands to have additional support for attacking either the pole or the base of an I.V. stand to a bed or wheelchair, etc. on which a patient rests while receiving intravenous injection. Furthermore, it is not unusual for doctors and nurses to hang additional loads such as medical instruments, devices or other articles on the post or pole of an I.V. stand. Therefore, the stability of such stands and the rigidity and strength of the post or pole of I.V. stands are particularly important. Another disadvantage of conventional I.V. stands lies in that a disassembled I.V. stand is usually packed in a relatively large box due to the relatively large sizes of the components, particularly the base unit. Therefore, it is also desirable to have an I.V. stand assembly which can be disassembled into a kit package having relatively small dimensions for shipping and storage.

### SUMMARY OF THE INVENTION

In accordance with one aspect of the present invention, an intravenous support apparatus is provided as claimed in claim 1.

In one embodiment, the legs may be substantially identical and the inner end of the respective legs may be integrated with a pair of side connectors, The side connectors of adjacent legs may be connected one to another in order to join the inner ends of the legs together.

In accordance with another aspect of the present invention, a kit of components is provided for assembly of the above described intravenous support apparatus.

### BRIEF DESCRIPTION OF THE DRAWINGS

Further features and advantages of the present invention will become apparent from the following detailed description, taken in combination with the appended drawings, in which:
Figure 1 is a perspective view of an intravenous stand according to one embodiment of the present invention;
Figure 2 is an exploded, perspective partial view of a pole of the intravenous stand of Figure 1, showing a joint member for joining upper and lower parts of a bottom section of the pole;
Figure 3 is a perspective bottom view of a disconnectable leg which is part of a base of the intravenous stand of Figure 1;
Figure 4 is a cross-sectional view of the leg of Figure 3 taken along 4-4, showing the two-piece configuration of the leg;
Figure 5 is a perspective view of a core element of the leg of Figure 4;
Figure 6 is a partial perspective bottom view of three legs of the intravenous stand of Figure 1 in an assembly procedure, showing the side connectors of adjacent legs for connection to each other;
Figure 7 is perspective bottom view of an assembled base of the intravenous stand of Figure 1, showing the legs connected one to another;
Figure 8 is a top plane view of a bottom plate used to cover the side connectors of the connected legs from a bottom side; and
Figure 9 is a schematic illustration of a box for containing a package of a kit to be assembled into the intravenous stand of Figure 1.

It will be noted that throughout the appended drawings, like features are identified by like reference numerals.

### DETAILED DESCRIPTION OF THE PREFERRED EMBODIMENT

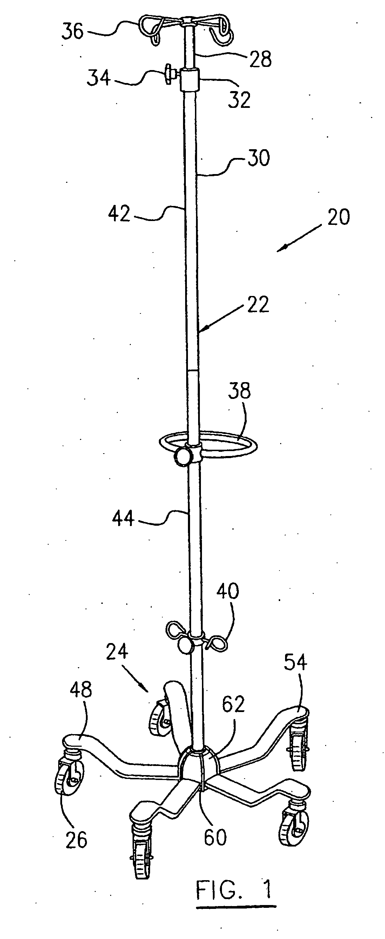

In Figure 1, an intravenous support apparatus generally referred to as an intravenous (I.V.) stand 20 includes a pole 22 in a substantially upright position, inserted into and supported by a base assembly 24 which preferably has a plurality of small wheels, such as wheel assemblies 26 for convenience of transportation.

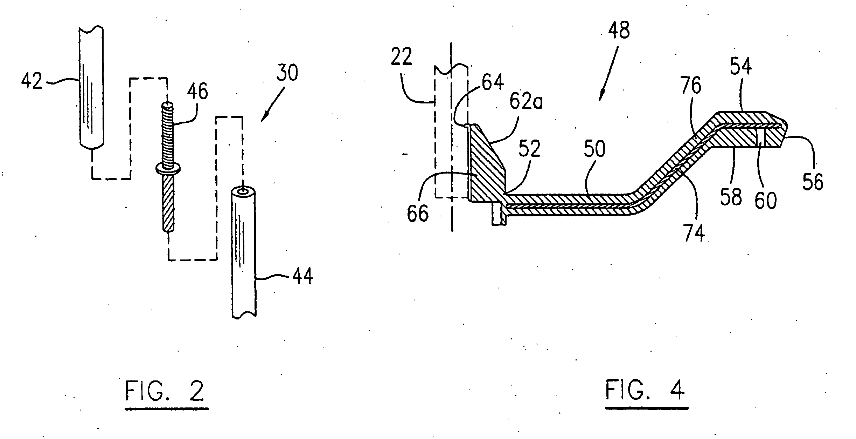

Referring to Figures 1 and 2, the pole 22, according to one embodiment of the present invention, may include a top section 28 and a bottom section 30 which is in a hollow configuration, such as of a steel tube. The top section 28 may also be made of a steel tube but has another diameter smaller than the inner diameter of the bottom section 30 so as to allow the top section 28 to be inserted into the bottom section 30 to form a telescoping configuration. Therefore, the top section 28 can be extended upwardly from or be retracted downwardly into the bottom section 30. A lock device may be provided to lock the top section 28 in a selected position with respect to the bottom section 30. The lock device for example may include a sleeve member 32 frictionally affixed on a top end of the bottom section 30 for receiving a locking screw 34 transversely extending through the sleeve member 32 to press against the top section 28 as it extends through the sleeve member 32.

Means are also provided for hanging intravenous bottles or pouches of intravenous liquids on the pole 22. For example, hooks 36 are removably attached to a top end of the top section 28 as shown in Figure 1. Optionally, other accessories such as a patient handle 38 and additional hooks 40 may be attached to the pole, preferably on the bottom section 30 in a height adjustable manner, which, for example, may be used to support an I.V. pump if needed.

In consideration of convenience for packaging, the pole 22, particularly the bottom section 30 thereof, may include separable upper and lower parts 42, 44, which are both made from, for example, a same steel tube, therefore having substantially equal outer diameters and having substantially equal inner diameters greater than the outer diameter of the top section 28. As more clearly illustrated in Figure 2, the upper part 42 and lower part 44 are joined end to end to form the bottom section 30 of the pole 20. A joint member 46 may be used to join the upper and lower parts 42, 44 together. The joint member 46, for example, includes a threaded bolt with a middle shoulder (not indicated). The shoulder preferably has an outer diameter substantially equal to the outer diameter of the upper and lower parts 42, 44 and the threading extends along the entire length of the bolt. The threads defined in the respective upper section and lower section of the bolt, which are separated by the middle shoulder, may have opposite rotational directions for convenience of engagement with complementary inner threads defined in the respective upper and lower parts 42, 44 of the bottom section 30. In such a configuration, the pole 20 can be conveniently disassembled into three tubular sections which may have roughly similar lengths for packaging, while a height adjustment can be easily achieved with the telescoping configuration between the top section 28 and the upper part 42 of the bottom section 30. In a conventional multiple telescoping configuration however, three or more sections of the pole are all connected in telescoping configurations and the diameter of the respective telescoping sections of the pole from the bottom to the top is progressively smaller one than another, which provides height adjustability but compromises the strength and rigidity of the pole and thus the load bearing capacity of the I.V. stand. The embodiment of this invention however maintains one telescoping configuration for convenience of height adjustment of the pole while providing a more secure but disconnectable joining structure between the upper and lower parts 42, 44 of the bottom section 30 of the pole, which advantageously and significantly increases the strength and rigidity of the pole 22 and thereby increases the lead bearing capacity of the intravenous stand 20.

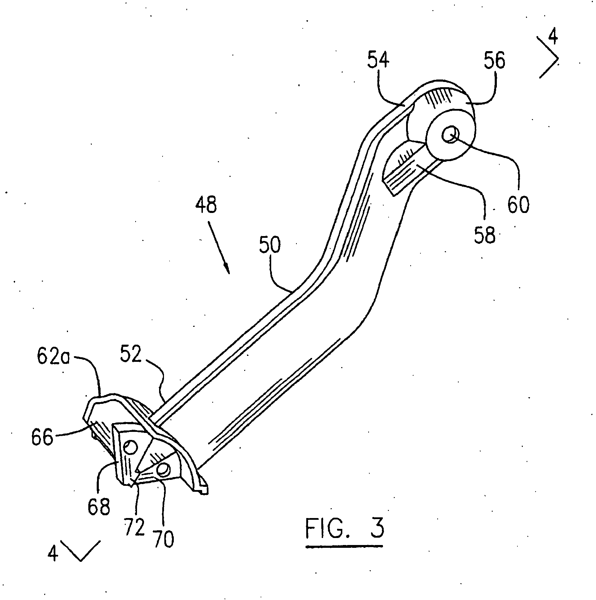

In Figure 1 and 3-8, the base assembly 24 of the I.V. stand 20 according to one embodiment includes a plurality of disconnectable legs 48 (five legs shown in this embodiment). Each leg 48 is configured, for example with a substantially flat body 50 with an inner end 52 and an outer end 54. The outer end 54 may have a boss 56 defining a bottom surface 58 and a hole 60 which extends inwardly and upwardly from the bottom surface 58 for receiving a shaft (not shown) of the small wheel assembly 26 when the wheel assembly 26 is attached to the outer end 54 of the leg 48, abutting the bottom surface 58.

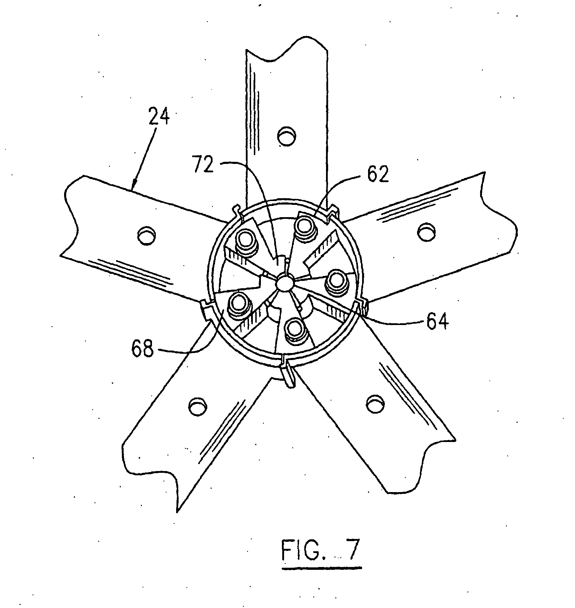

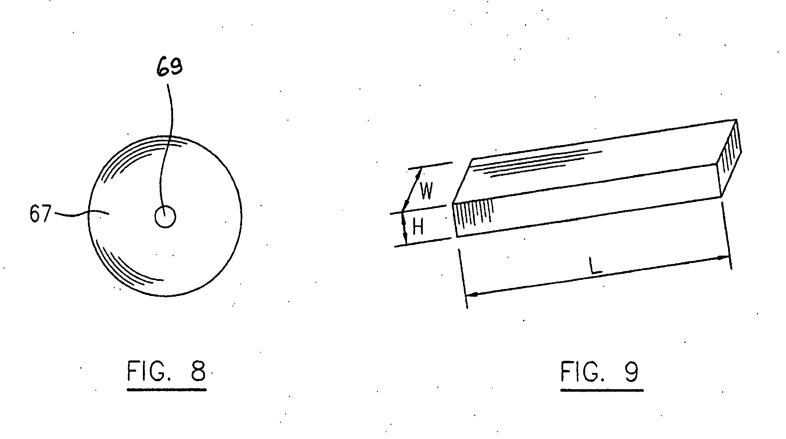

The inner ends 52 of the respective legs 48 join one to another to form a central body 62 (see Figure 1) of the base assembly 24 and to define a central passage 64 (see Figure 7) extending substantially vertically through the base assembly 24. The central passage 64 receives a bottom end of the pole 22, for example the bottom end of the lower part 44 of the bottom section 30 of the pole, for supporting the pole 22 in an upright position as shown in Figure 1. The bottom end of the pole 22 is secured within the central passage 64 of the central body 62 using a bottom plate 67 (see figure 8) attached to the bottom side of the central body 62 and a screw fastener (not shown) inserted through a central passage 69 of the plate 67. The screw fastener is securely engaged with the inner thread of the bottom end of the pole 22 (the bottom end of the lower pan 44 of the bottom section 30 of the pole 22 in this embodiment). The substantially flat body 50 of the respective legs 48 may be bent such that the central body 62 formed with the respective inner ends 52 of the legs 48 is positioned at a level which is lower than a height level of the outer ends 54 of the legs 48 when the I.V. stand 20 is standing upright on the ground or floor. Therefore, when the outer ends 54 of the legs 48 are supported by the respective small wheel assemblies 26 at a relatively higher level with respect to the ground, the central body being in a relatively lower position, advantageously increases the stability of the I.V. stand 20 when the pole 20 is loaded.

Each of the inner ends 52 of the respective legs 48, is integrated with a sectorial fraction 62a of the central body 62 of the base assembly 24 (1/5 of the central body 62 in this embodiment). The sectorial fraction 62a is configured in a shell configuration, and projects upwardly from the inner end 52 of the leg 48, thereby forming an upwardly projecting profile of the central body 62 when the five legs 48 are assembled together to form the base assembly 24. The upwardly projecting profile of the central body 62 provides a vertical dimension for more effectively securing the bottom end of the pole 22. The shell configuration of the sectorial fraction 62a of each leg 48 may include a central wall 66 to increase the rigidity of the hollow configuration of the central body 60 formed by the individual shell configurations of the sectorial fractions 62a of the legs 48.

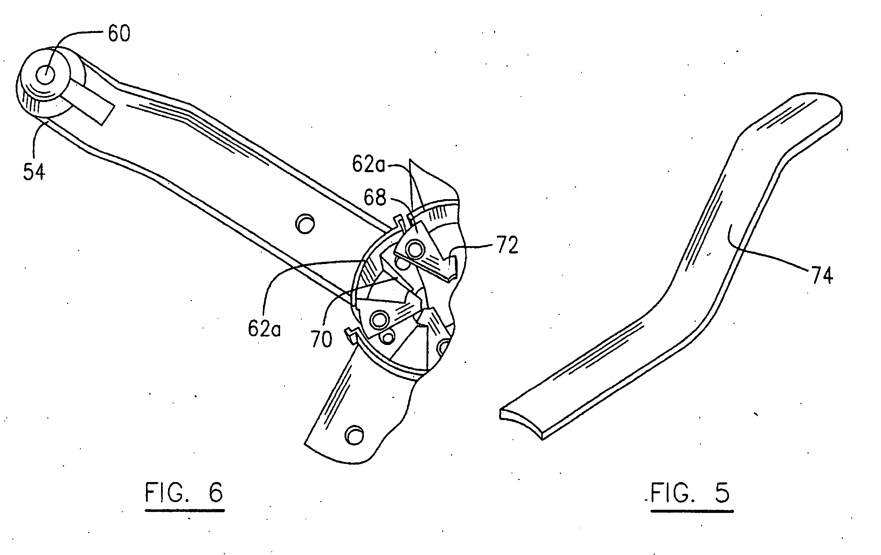

The inner end 52 of each leg 48 may be incorporated with a pair of side connectors 68 and 70 which are adapted to be positioned adjacent one another and to be connected when the respective legs 48 are assembled together, thereby joining the inner ends 52 of the legs 48 and securing the respective sectorial fractions 62a in position to form the complete central body 62. The respective side connectors 68 and 70 of each leg 48 are configured substantially in a sectorial configuration and are affixed to the inner end 52 of the leg 48 in locations offset one to another both in circumference and in height with respect to the shell configuration of the sectorial fraction 62a. For example, side connector 68 projects circumferentially out of the shell configuration of the sectorial fraction 62a and is adapted to be received within the adjacent shell configuration of sectorial fraction 62a when the legs 48 are assembled together (see Figure 6). Side connector 70, however, is substantially located within the shell configuration of sectorial fraction 62a at the other side thereof and at a relatively higher location with respect to the ground when the I.V. stand is positioned upright on the ground or floor, so as to match a corresponding side connector 68 of an adjacent leg 48 which extends into a space below the side connector 70 when the adjacent leg 48 is positioned at the other side of said leg 48. A hole (not indicated) extends through the respective side connectors 68 and 70 so that the hole in one side connector 68 will align with the hole in a adjacent side connector 70, when all the legs are assembled in position as shown in Figure 7. Screws (not indicated) are inserted from the bottom side of the central body 62 into the aligned holes to engage with inner threads defined in the hole of the side connector 70 (the connector in the higher position), thereby securing the side connectors of adjacent legs 48 together.

The central body 62 formed with the sectorial fraction 62a in the shell configuration, substantially houses the side connectors 68, 70 of the respective legs 48 when assembled so that the side connectors 68, 70 are not visible from a top view of the I.V. stand 20. The side connectors 68, 70 are also not visible from a bottom view of the I.V. stand when the plate 67 is attached to the bottom side of the central body 62.

It should be noted that the substantially sectorial side connector 68 may further have an enlarged tip portion 72 (see Figure 7) which is configured to function as a positioning element to provide convenience of angularly positioning the legs 48 for the assembly procedure and to substantially define a bottom opening of the central passage 46. A top opening of the central passage 64 is also defined by the shell configuration of the sectorial fractions 62a in combination, when the legs 48 are assembled together. The top and bottom openings of the central passage 64 of the central body 62 have an inner diameter slightly greater than the outer diameter of the bottom section 30 of the pole 22 in order to ensure an appropriate fit when the bottom section 30 of the pole 22 is inserted into the base assembly 24. The center wall 66 in the shell configuration of the respective sectorial fractions 62a is configured to not interfere with the insertion of the bottom end 30 of the pole 22, as more clearly shown in Figure 4. Optionally, the bottom opening of the central passage 64 formed by the enlarged tip portions 72 of the sectorial side connectors 68, may have a diameter smaller than the outer diameter of the bottom section 30 of the pole 22 to prevent the pole 22 from being over-inserted into the base assembly 24, but allowing the fastener to pass therethrough to secure the pole 22 to the base assembly 24.

Each leg 48 may be of an integral two-piece configuration having a first material containing a core element of a second material which is heavier and stronger than the first material. According to one embodiment as shown in Figure 4, the leg 48 may include a steel band 74 bent in a configuration desirable for the leg 48, as shown in Figure 5. An outer layer composed of a second material, for example an aluminium alloy outer layer 76, is formed in a molding process to completely surround the steel band 74 in order to form the desired leg configuration. This integral two-piece configuration advantageously provides all the advantages of an aluminium alloy to a base of an I.V stand including an aesthetically pleasing appearance, convenience of manufacturing, resistance to rust etc., while overcoming the disadvantage of lacking stability because the aluminium alloy is relatively light and unable to sustain heavy loads in comparison with steel.

The further advantage of the I.V. stand according to the embodiments of this invention, lies in that the I.V. stand 20 can be completely disassembled and packaged into a relatively compact kit of components as described above, in contrast to the package size for conventional I.V. stand assemblies. Hospitals often purchase and store large quantities of I.V. stand assemblies. The problem with conventional products is that the pole cannot be collapsed to less than a length of 4.5 feet (1.37 m) and the base which is typically manufactured as a complete unit cannot be disassembled and is therefore bulky and unwieldy, The larger size of the packages for such conventional I.V. stands adds to the cost of shipping and requires larger storage space and as such poses a significant problem. The I.V. stand 20 can be provided in a kit package contained in a rectangular box as shown in Figure 9, having the smaller and more compact dimensions L x W x H wherein L is between 34.5 inches (0.88 m) and 34 inches (0.86 m), W is between 6 inches (0.15 m) and 5.5 inches (0.14 m) and H is between 5 inches (0.13 m) and 4.5 inches (0.11 m), which reduces shipping costs and takes up much less storage space.

The above description is meant to be exemplary only and one skilled in the art will recognize that changes may be made to the embodiments described without departure from the scope of the invention disclosed. For example, the core element and the surrounding outer layer of the legs may be made with materials other than steel and aluminium alloy, such as iron and plastic, etc. The connectors of the respective legs may be configured differently from the above-described configurations to similarly achieve a direct connection of adjacent legs without a separate connector, as the described embodiment does. Still other modifications which fall within the scope of the present invention will be apparent to those skilled in the art in light of a review of this disclosure and such modifications are intended to fall within the appended claims.

## Claims

1. An intravenous support apparatus (20) comprising:
a pole (22);
means (36) attachable to the pole (22) for hanging an intravenous liquid supply on the pole (22); and **characterised by**:
a base (24) including a plurality of disconnectable legs (48) having respective inner ends (52) joined one with another, the joined inner ends (52) forming a central body (62) of the base (24) to define a central passage (64) extending vertically through the base (24), the central passage (64) receiving a bottom end of the pole (22) for supporting the pole (22) in a upright position, each leg (48) including a first material (76) containing a core element (74) of a second material which is heavier than the first material;
wherein the inner end (52) of the respective legs (48) is integrated with a sectorial fraction (62a) of the central body (62) of the base (24), the sectorial fraction (62a) of each leg (48) being of a shell configuration and projecting upwardly from the inner end (52) of the leg (48), thereby forming an upwardly projecting profile of the central body (62) of the base (24) in order to provide a vertical dimension for securing the bottom end of the pole (22).

2. The intravenous support apparatus as defined in claim 1 wherein the base (24) comprises a plurality of wheels (26) each attached to an outer end (54) of the respective legs (48).

3. The intravenous support apparatus as defined in claim 2 wherein the legs (48) are bent such that the central body (62) formed with the inner ends (52) of the
respective legs (48) is positioned lower than the outer ends (54) of the respective legs (48),

4. The intravenous support apparatus as defined in any preceding claim wherein the legs (48) are substantially identical, the inner end (52) of the respective legs (48) being integrated with a pair of side connectors (68, 72), the side connectors (68, 72) of adjacent legs (48) being connected one to another in order to join the inner ends (52) of the legs (48) together.

5. The intravenous support apparatus as defined in any preceding claim wherein the first material of the leg (48) is aluminum.

6. The intravenous support apparatus as defined in any preceding claim wherein the second material of the core element (74) of the leg (48) is steel.

7. The intravenous support apparatus as defined in any of claims 1 to 4 wherein each of the legs (48) comprises a steel band (74) as the core element surrounded by molded aluminum (76).

8. The intravenous support apparatus as defined in any preceding claim wherein the pole (72) comprises a top section (28) having an outer diameter and a bottom section (30) having an inner diameter greater than the outer diameter of the top section (28), the top section (28) being adjustably connected to the bottom section (30) in a telescoping configuration.

9. The intravenous support apparatus as defined in claim 8 wherein the bottom section (30) of the pole (22) comprises an upper part (42) and a lower part (44), both having the inner diameter of the bottom section (30), the upper and lower parts (42, 44) being disconnectable one from another.

10. The intravenous support apparatus as defined in claim 9 wherein the upper and lower parts (42, 44) of the bottom section (30) of the pole (22) are tubular to
allow a joint member (46) to join the upper and lower parts (42, 44) together by insertion into an inside of both the upper and lower parts (42, 44).

11. A kit of components for an assembly of the intravenous support apparatus as defined in any one of claims 1 to 10.

12. The kit as defined in claim 11 wherein the all components of the kit are configured and sized to allow being packed within a package size not greater than 0.88 m x 0.15 m x 0.14 m.

## Patentansprüche

1. Intravenöse Stützvorrichtung (20) mit einer Stange (22),
an der Stange (22) anbringbaren Mitteln (36) zum Aufhängen eines Vorrats an intravenöser Flüssigkeit an der Stange (22),
**gekennzeichnet durch**
eine Basis (24) mit mehreren abnehmbaren Beinen (48) mit jeweiligen miteinander verbundenen inneren Enden (52), wobei die verbundenen inneren Enden (52) einen mittleren Körper (62) der Basis (24) bilden, um einen mittleren Durchgang (64) zu definieren, der sich vertikal **durch** die Basis (24) erstreckt, wobei der mittlere Durchgang (64) ein unteres Ende der Stange (22) aufnimmt, um die Stange (22) in einer aufrechten Position zu stützen, wobei jedes Bein (48) ein erstes Material (76) aufweist, das ein Kernelement (74) eines zweiten Materials enthält, das schwerer als das erste Material ist,
wobei das innere Ende (52) der jeweiligen Beine (48) mit einem Sektorteil (62a) des mittleren Körpers (62) der Basis (24) integriert ist, wobei der Sektorteil (62a) jedes Beins (48) eine Schalenkonfiguration hat und vom inneren Ende (52) des Beins (48) nach oben vorragt und **dadurch** ein nach oben vorragendes Profil des mittleren Körpers (62) der Basis (24) bildet, um eine vertikale Dimension zur Befestigung des unteren Endes der Stange (22) bereitzustellen.

2. Intravenöse Stützvorrichtung nach Anspruch 1, wobei die Basis (24) mehrere Räder (26) umfasst, die jeweils an einem äußeren Ende (54) der jeweiligen Beine (48) angebracht sind.

3. Intravenöse Stützvorrichtung nach Anspruch 2, wobei die Beine (48) so gebogen sind, dass der mit den inneren Enden (52) der jeweiligen Beine (48) gebildete mittlere Körper (62) niedriger als die äußeren Enden (54) der jeweiligen Beine (48) positioniert ist.

4. Intravenöse Stützvorrichtung nach einem der vorhergehenden Ansprüche, wobei die Beine (48) im Wesentlichen identisch sind, wobei das innere Ende (52) der jeweiligen Beine (48) mit einem Paar Seitenverbindern (68, 72) integriert ist, wobei die Seitenverbinder (68, 72) benachbarter Beine (48) miteinander verbunden sind, um die inneren Enden (52) der Beine (48) miteinander zu verbinden.

5. Intravenöse Stützvorrichtung nach einem der vorhergehenden Ansprüche, wobei das erste Material des Beins (48) Aluminium ist.

6. Intravenöse Stützvorrichtung nach einem der vorhergehenden Ansprüche, wobei das zweite Material des Kernelements (74) des Beins (48) Stahl ist.

7. Intravenöse Stützvorrichtung nach einem der Ansprüche 1 bis 4, wobei jedes der Beine (48) ein Stahlband (74) als das von geformtem Aluminium (76) umgebene Kernelement umfasst.

8. Intravenöse Stützvorrichtung nach einem der vorhergehenden Ansprüche, wobei die Stange (72) einen oberen Abschnitt (28) mit einem äußeren Durchmesser und einen unteren Abschnitt (30) umfasst, dessen innerer Durchmesser größer als der äußere Durchmesser des oberen Abschnitts (28) ist, wobei der obere Abschnitt (28) verstellbar mit dem unteren Abschnitt (30) teleskopartig verbunden ist.

9. Intravenöse Stützvorrichtung nach Anspruch 8, wobei der untere Abschnitt (30) der Stange (22) einen oberen Teil (42) und einen unteren Teil (44) umfasst, die beide den inneren Durchmesser des unteren Abschnitts (30) haben, wobei der obere und der untere Teil (42, 44) voneinander getrennt werden können.

10. Intravenöse Stützvorrichtung nach Anspruch 9, wobei der obere und der untere Teil (42, 44) des unteren Abschnitts (30) der Stange (22) rohrförmig sind, damit der obere und der untere Teil (42, 44) über ein Gelenkelement (46) miteinander verbunden werden können, das in ein Inneres sowohl des oberen als auch des unteren Teils (42, 44) eingeführt wird.

11. Satz aus Komponenten für eine Anordnung der intravenösen Stützvorrichtung nach einem der Ansprüche 1 bis 10.

12. Satz nach Anspruch 11, wobei alle Komponenten des Satzes so konfiguriert und bemessen sind, dass sie in einer Packungsgröße von höchstens 0,88 m x 0,15 m x 0,14 m verpackt werden können.

## Revendications

1. Appareil de support intraveineux (20), comprenant :
un poteau (22) ;
un moyen (36) pouvant être attaché au poteau (22) pour accrocher une source de liquide intraveineux au poteau (22) ; et **caractérisé par** :
une base (24) comportant une pluralité de pieds déconnectables (48) ayant des extrémités internes respectives (52) réunies les unes aux autres, les extrémités internes réunies (52) formant un corps central (62) de la base (24) pour définir un passage central (64) s'étendant verticalement à travers la base (24), le passage central (64) recevant une extrémité inférieure du poteau (22) pour supporter le poteau (22) dans une position debout, chaque pied (48) comportant un premier matériau (76) contenant un élément de coeur (74) en un deuxième matériau qui est plus lourd que le premier matériau ;
l'extrémité interne (52) des pieds respectifs (48) étant intégrée à une fraction sectorielle (62a) du corps central (62) de la base (24), la fraction sectorielle (62a) de chaque pied (48) ayant une configuration en forme de coque et saillant vers le haut depuis l'extrémité interne (52) du pied (48), pour ainsi former un profilé saillant vers le haut du corps central (62) de la base (24) afin de fournir une dimension verticale pour fixer l'extrémité inférieure du poteau (22).

2. Appareil de support intraveineux selon la revendication 1, dans lequel la base (24) comprend une pluralité de roulettes (26), chacune étant attachée à une extrémité externe (54) des pieds respectifs (48).

3. Appareil de support intraveineux selon la revendication 2, dans lequel les pieds (48) sont recourbés de telle sorte que le corps central (62) formé avec les extrémités internes (52) des pieds respectifs (48) soit positionné plus bas que les extrémités externes (54) des pieds respectifs (48).

4. Appareil de support intraveineux selon l'une quelconque des revendications précédentes, dans lequel les pieds (48) sont substantiellement identiques, l'extrémité interne (52) des pieds respectifs (48) étant intégrée à une paire de connecteurs latéraux (68, 72), les connecteurs latéraux (68, 72) de pieds adjacents (48) étant connectés les uns aux autres afin de réunir les extrémités internes (52) des pieds (48) les unes aux autres.

5. Appareil de support intraveineux selon l'une quelconque des revendications précédentes, dans lequel le premier matériau du pied (48) est de l'aluminium.

6. Appareil de support intraveineux selon l'une quelconque des revendications précédentes, dans lequel le deuxième matériau de l'élément de coeur (74) du pied (48) est de l'acier.

7. Appareil de support intraveineux selon l'une quelconque des revendications 1 à 4, dans lequel chacun des pieds (48) comprend une bande d'acier (74) en tant qu'élément de coeur, entourée par de l'aluminium moulé (76).

8. Appareil de support intraveineux selon l'une quelconque des revendications précédentes, dans lequel le poteau (72) comprend une section supérieure (28) ayant un diamètre extérieur et une section inférieure (30) ayant un diamètre intérieur supérieur au diamètre extérieur de la section supérieure (28), la section supérieure (28) étant connectée de manière ajustable à la section inférieure (30) dans une configuration télescopique.

9. Appareil de support intraveineux selon la revendication 8, dans lequel la section inférieure (30) du poteau (22) comprend une partie supérieure (42) et une partie inférieure (44), toutes deux ayant le diamètre intérieur de la section inférieure (30), les parties supérieure et inférieure (42, 44) pouvant être déconnectées l'une de l'autre.

10. Appareil de support intraveineux selon la revendication 9, dans lequel les parties supérieure et inférieure (42, 44) de la section inférieure (30) du poteau (22) sont tubulaires, de manière à permettre à un organe de joint (46) de réunir les parties supérieure et inférieure (42, 44) l'une à l'autre par l'insertion à l'intérieur à la fois des parties supérieure et inférieure (42, 44).

11. Kit de composants pour l'assemblage de l'appareil de support intraveineux selon l'une quelconque des revendications 1 à 10.

12. Kit selon la revendication 11, dans lequel tous les composants du kit sont configurés et dimensionnés de manière à permettre leur emballage dans un emballage d'une dimension ne dépassant pas 0,88 m x 0,15 m x 0,14 m.
